# EUROPEAN PATENT APPLICATION

(11) **EP 4 830 615 A1**
(43) Date of publication of application: **28.10.2026**
(21) Application number: 26165259.8
(22) Date of filing: 17.03.2026
(51) Int. Cl.: A61B 1/015, A61B 1/018, A61B 1/07, A61B 1/00

(54) **WORKING CHANNEL AND SURGICAL INSTRUMENT**

(30) Priority: 25.04.2025 LU 601325
(71) Applicant: Karl Storz SE & Co. KG, 78532 Tuttlingen (DE)
(72) Inventor: TOMBERG, Johan, 76401 Laagri (EE)

(57) **Abstract**

The present invention provides a working channel (1) configured for communicating fluids or medical tools through a shaft (10) of a surgical instrument (100), in particular of a flexible endoscopic instrument, comprising: a tubular body (2) with a main canal (3) for receiving the fluids or medical tools and with a secondary canal (4); and an optical fiber (5), which is integrated in the secondary canal (4). Further the present invention provides a surgical instrument (100) comprising such a working channel (1).

## Description

### TECHNICAL FIELD

The present invention pertains to a working channel configured for communicating fluids or medical tools through a shaft of a surgical instrument, in particular of a flexible endoscopic instrument. Furthermore, the present invention pertains to a surgical instrument with such a working channel.

### BACKGROUND

Flexible surgical tube shaft instruments such as flexible endoscopes are used for a variety of applications in medicine and technology. Such flexible endoscopes comprise a flexible elongated shaft which is suitable for insertion into a cavity, such as an internal body cavity or a cavity of a technical object. As a rule, an endoscope lens is arranged at the tip of the endoscope shaft to generate an image of a scene in the observed cavity. To record and transmit the endoscopic image from the distal (i.e. far from the observer) to the proximal (i.e. close to the observer) end region of the endoscope, an ordered bundle of optical fibers running inside the shaft can be provided, for example, or an electronic image sensor, such as a CCD chip, which is arranged in the region of the distal end of the shaft and whose signals are transmitted to the proximal end region via electrical lines running inside the shaft. Since there is usually not enough light in the observed cavity, a light guide system can also be arranged inside the shaft to transport light to the distal end of the endoscope, where it is used to illuminate the cavity. Further, the endoscope shaft may include one or more working channels for passing endoscopic working instruments from the proximal to the distal end portion of the shaft for performing manipulations within the cavity.

Flexible surgical tube shaft instruments are also known which comprise a flexible elongated shaft which is also suitable for insertion into a cavity, such as an internal body cavity or a cavity of a technical object. Such a flexible endoscopic instrument can be used to carry out manipulations in the cavity and for this purpose can be designed, for example, as a grasping instrument for grasping and manipulating tissue or objects in the cavity inside the body or in the cavity of a technical object. For this purpose, a tool is arranged at the distal end of the flexible shaft, which can be operated from the proximal end of the shaft via a transmission means running inside the shaft. Such a flexible endoscopic instrument usually does not have its own optics for recording an endoscopic image, but can be used in particular together with a flexible endoscope.

Known endoscopes either have a LED placed next to the camera unit in the distal head of the endoscope, in particular in most single-use endoscopes or small reusable endoscopes, or have a light-guiding cable to transmit light to the distal head, wherein the light source is in the handle of the endoscope or in the supply unit connector. Such a light-guiding cable is present in most reusable endoscopes.

However, as the LED's and light bundles are taking up valuable space in the endoscope insertion tip other solutions are necessary. In some prior art solutions separate light fibers or bundles are provided, but they are fragile by themselves and need additional elastomer cover materials in order to ensure mechanical durability, e.g. silicone tubing over the fibers.

### DISCLOSURE OF THE INVENTION

It is an object of the present invention to provide a working channel, which can simplify and miniaturize endoscope designs keeping in mind low cost and usability.

According to the invention, this problem is solved by a flexible shaft for a surgical instrument with the features of claim 1, by a surgical instrument with the features of claim 8, a method for manufacturing a flexible shaft for a surgical instrument with the features of claim 11 and/or by a method for assembling a steering wire to a flexible shaft for a surgical instrument with the features of claim 13.

According to a first aspect of the invention, a working channel configured for communicating fluids or medical tools through a shaft of a surgical instrument, in particular of a flexible endoscopic instrument, is provided. The working channel comprises a tubular body with a main canal for receiving the fluids or medical tools. The tubular body also has a secondary canal. The working channel further comprises an optical fiber, which is integrated in the secondary canal.

According to a second aspect of the invention, a surgical instrument, in particular flexible endoscopic instrument, is provided. The surgical instrument comprises a shaft with a distal end and a proximal end. Furthermore, the surgical instrument comprises a working channel according to the first aspect of the invention. The working channel is insertable into the shaft. The surgical instrument further comprises a light source, which is connected to a proximal end of the working channel and transmits light to the optical fiber.

A fundamental concept of the invention is to provide the endoscope working channel with the light conducting fiber respectively optical fiber in order to transfer illumination light or data signal for an endoscope camera unit respectively optical image device. The tubular body is not limited to cylindrically shaped tubes, but can also form an oval shaped tube, a tube with a bulge or similar shapes. In other words, the tubular body can have an irregular outer shape. Thereby, the main canal respectively tunnel or the secondary canal respectively tunnel or both can have a constant diameter, in particular a circular cross-section.

The present invention can be applied to all flexible endoscopes that have a camera unit and need a light source in order to have a visible video image. A distal end of the working channel corresponds to the distal end of the shaft or surgical instrument.

A particular advantage in the solution according to an aspect of the invention is that the optical fiber is made of a more rigid material than the tubular body. Thus, the optical fiber can reinforce the tubular body or the working channel, respectively.

A further advantage of the present invention is that a process automation or an assembly automation can be optimized. An assembly process can be simplified as there are less components to assemble inside the shaft. Hence, a diameter of the distal end of the surgical instrument can be significantly reduced. In particular, the light source can be directly connected to a proximal end of the optical fiber. The light source can be configured as a LED.

A distal head, which can be mounted at the distal end, can have a smaller dimension or can be omitted completely. The distal head may comprise less connectors since a light source does not need to be arranged at the distal head. The optical fiber has a smaller cross section than the light source and is integrated in the working channel.

The operating interface and/or the working channel can be made of a plastic material. For example, the working channel can be completely made of a plastic material, in particular when the surgical instrument is intended to use only once.

The working channel can be inserted into the shaft of the surgical instrument. The working channel is mounted or guided at least at an end, in particular an proximal end, of a rod in the surgical instrument. In particular, the rod is mounted or guided in the shaft in such a way that the working channel cannot move or rotate axially relative to the tube shaft.

A medical tool, which can be designed in particular in the form of an interchangeable accessory, can be fluidly coupled to the operating interface. For example, the medical tool can be configured as pliers, scissors, a gripper or the like.

If the surgical instrument is to be operated by a user such as a surgeon, the surgical instrument comprises a handle as a grip for the user. The operating interface then transmits a force or torque, which is applied by the user to the handle via a suitable actuation (e.g. movable handle leg), to a force transmission element for example.

If the surgical instrument is to be connected to a robot, the surgical instrument comprises a corresponding connection interface for the robot, which can apply an axial force or torque to the force transmission element via the operating interface.

Advantageous embodiments and further developments emerge from the description with reference to the figures.

According to some embodiments of the invention, the working channel is manufactured in one manufacturing process, in particular in one extrusion process. Hence, manufacturing can be quicker. That means, the optical fiber respectively light conducting fiber is already placed in the tubular body during the one manufacturing process. In particular, the working channel can be configured as one piece, which is manufactured by one extrusion process. Furthermore, a working channel for single use endoscope can be manufactured with extrusion.

Alternatively, the tubular body can comprise a multilayer wall, wherein the optical fiber is embedded between two layers of the multilayer wall. Hence, the working channel can provide a combination of two components that have different purposes, namely the optical fiber for illumination and the tubular body for guiding the fluids or medical tools, into one single part.

According to some further embodiments of the invention, the optical fiber is made of a glass material or of a transparent plastic material. The transparent plastic material can be polymethylmethacrylat, short PMMA, for example.

According to some further embodiments of the invention, the optical fiber has a cladding, that is configured to reflect and guide the light in the optical fiber. When embedded within the working channel, a channel wall respectively the tubular body can protect the optical fiber creating a very compact structure. The cladding can be arranged on an outer surface of the optical fiber, for example.

According to some further embodiments of the invention, the optical fiber is arranged flush with a distal end of the tubular body. With this arrangement a fiber end of the optical fiber can distribute light from the distal end of the tubular body.

According to some further embodiments of the invention, the secondary canal comprises a plurality of secondary canals, and wherein the optical fiber comprises a to that corresponding plurality of optical fibers, wherein each optical fiber is integrated in one of the plurality of secondary canals. That means, the plurality of secondary canals comprises the secondary canal as described above and further secondary canals. The same applies to the plurality of optical fibers. The plurality of secondary canals and the plurality of optical fibers may each comprise two, three, four, five or six secondary canals and optical fibers, respectively.

According to some further embodiments of the invention, the optical fiber is helically arranged around the main canal. The helical construction of the optical fiber allows the working channel to be more flexible or elastically deformable during its use. Additionally, light can be distributed in a wider field of view respectively illumination range when the working channel comprises the plurality of optical fibers as each optical fiber is directed at a different angle.

According to some further embodiments of the invention, an outer shell surface forms a groove, which extends parallel to the secondary canal. The groove can be configured to secure a position of a camera head and its cables along the working channel. Thus, a more simple and easier to assemble construction of the working channel can be provided. For example, the groove can have a rectangular shape, a curved shape or combinations thereof.

According to some further embodiments of the invention, the groove is formed between two secondary canals. Thus, the irregular outer shape created by the plurality of secondary canals enables the groove that houses the camera head and/or electrical cables or similar features.

Optionally, the working channel can house a combination of the optical fiber and an electrical conductor embedded in-side the working channel.

According to some further embodiments of the invention, a distal end of the working channel is arranged flush with a distal end of the surgical instrument. Hence, light can be distributed inside a patient by the optical fiber.

According to some further embodiments of the invention, the shaft is configured as a flexible shaft for being articulatable at least in one plain.

According to some further embodiments of the invention, the surgical instrument further comprises an operating interface, which is arranged at the proximal end of the shaft and configured to operate the shaft. For example, the working channel is configured for communicating fluids or medical tools from the operating interface to the distal end of the shaft. Furthermore, a light-adapter component can be arranged in between the optical fiber and the LED, in particular when the LED is larger than the optical fiber.

For example, a number of light sources, in particular LEDs, can correspond to a number of optical fibers.

According to some further embodiments of the invention, the surgical instrument further comprises two steering wires extending from the operating interface through the flexible shaft for articulating the flexible shaft. For example, the surgical instrument can also comprise further two steering wires, so in total four steering wires.

According to some further embodiments of the invention, the surgical instrument further comprises an optical image device arranged at the distal end and extending from the operating interface along the working channel for generating image data of an area in front of the distal end.

Optionally, the distal end of the surgical instrument respectively insertion tip can comprise or be made out of a transparent material and act as an illumination source for the optical image device.

The above embodiments and further developments can be combined with one another arbitrarily, as far as appropriate. Further possible configurations, developments and implementations of the invention are also combinations of features of the invention described above or below for the exemplary embodiments that are not explicitly cited. In particular, a person skilled in the art will also add individual aspects as improvements or additions to the respective basic form of the present invention.

### BRIEF DESCRIPTION OF THE DRAWINGS

The present invention is explained more specifically below on the basis of the exemplary embodiments indicated in the schematic figures, in which:
Fig. 1 shows a handheld surgical instrument, in particular a steerable flexible endoscopic instrument, in an overall view according to an embodiment of the invention;
Fig. 2 shows a schematic cut view of an operating interface of the surgical instrument of Fig. 1;
Fig. 3 shows a schematic perspective view of a distal end of a working channel in combination with an optical image device according to a further embodiment of the invention;
Fig. 4 shows a schematic perspective view of the distal end of the working channel of Fig. 3;
Fig. 5 shows a schematic perspective view of a distal end of a working channel according to a further embodiment of the invention; and
Fig. 6 shows a cut view of a cross-section of the working channel of Fig. 5.

The accompanying figures are intended to convey a further understanding of the embodiments of the invention. They illustrate embodiments and are used in conjunction with the description to explain principles and concepts of the invention. Other embodiments and many of the cited advantages emerge in light of the drawings. The elements of the drawings are not necessarily shown to scale in relation to one another. Direction-indicating terminology such as for example "at the top", "at the bottom", "on the left", "on the right", "above", "below", "horizontally", "vertically", "at the front", "at the rear" and similar statements are merely used for explanatory purposes and do not serve to restrict the generality to specific configurations as shown in the figures.

In the figures of the drawing, elements, features and components that are the same, have the same function and have the same effect are each provided with the same reference signs - unless explained otherwise.

### DETAILED DESCRIPTION OF DRAWINGS

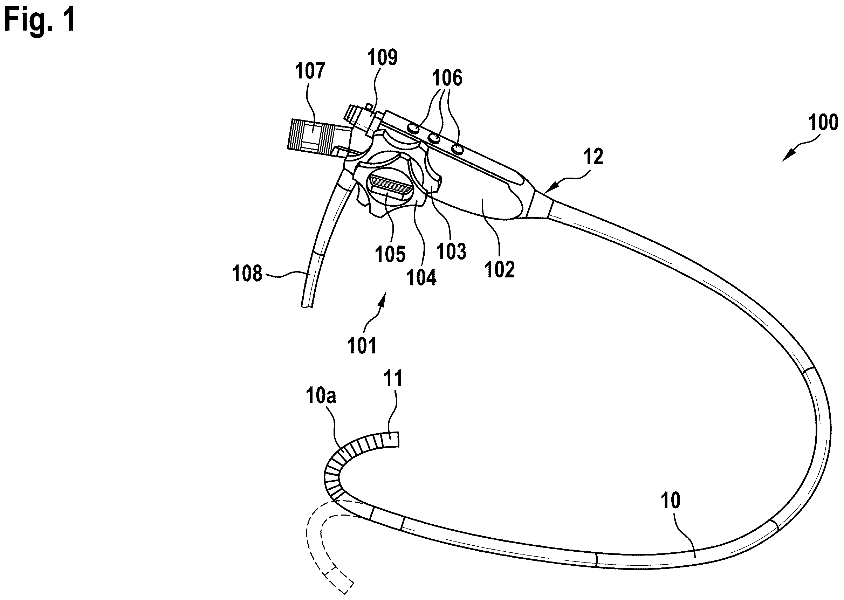

Fig. 1 shows an embodiment of a handheld surgical instrument 100, in particular of a steerable flexible endoscopic instrument 100, in an overall view.

As shown schematically in Fig. 1, the flexible endoscopic instrument 100 typically comprises a handpiece 101 and a shaft 10, the handpiece 101 being attached to a proximal end 12 of the shaft 10. The handpiece 101 can have an operating interface 102 made of a plastic and/or metallic material. On a lower side of the operating interface 102 a first hand wheel 103 and a second hand wheel 104 are arranged for controlling a deflection of a steerable section 10a of the shaft 10, as is described below. Typically, the first and the second hand wheel 103, 104 are arranged coaxially, and at an exterior side of the second hand wheel 104 a knob 105 for controlling a deflection brake relating to the second hand wheel 104 is provided. Further, the handpiece 101 may exhibit a multiplicity of control buttons 106 for controlling various functions of the flexible endoscopic instrument 100, such as for controlling the imaging and/or illumination system and/or irrigation and suction pumps, for example. The handpiece 101 may be connectable to an external video unit or a video monitor via connector 107 and to an external light source via light cable 108. Moreover, an instrument port 109 may be provided for inserting endoscopic instruments to be advanced through one or more respective channels to a distal end 11 of the shaft 10 for manipulating tissue or other objects within a cavity into which the shaft 10 can be inserted.

At its distal end 11 the shaft 10 comprises a steerable section 10a. The steerable section 10a may form a distal end section of the shaft 10 or, as shown in Fig. 1, may carry a distal end cap 11, which may accommodate an imaging optics and an electronic image sensor for providing an endoscopic image of a cavity into which the shaft 10 is inserted. The image signal generated by the image sensor may be transmitted via electric cables extending through the shaft 1 and the handpiece 101 to the connector 107 for being processed and displayed by an external video unit. The shaft 10 in total is flexible to an extent to be advanced through an endoscopic access or a hollow organ towards a cavity to be observed, being capable of adapting to a curved shape of the access or the organ. The steerable section 10a, on the other hand, is capable of being flexed actively by turning the hand wheels 103, 104. To this end the steerable section 10a comprises an inner structure of a plurality of shaft sections, thus being deflectable or articulatable in one or more planes. The plurality of shaft sections can be covered by a flexible tube to form a smooth outer surface, the shaft 10 in total having a uniform cross-sectional shape and diameter.

For controlling the deflection of the plurality of shaft sections two counteracting control wires (not shown in Fig. 1) are provided extending on opposite sides within the plurality of shaft sections, by a longitudinal movement of which the steerable section 10a can be bent to one or the other side, as indicated symbolically in Fig. 1. The control wires extend along the shaft 10 and are connected at their proximal ends to a deflection control mechanism arranged within the handpiece 101, which can be operated by a user by turning the hand wheels 103, 104 for deflecting the steerable section 10a. In the exemplary embodiment shown, the steerable section can be bent or articulated within a first plane that corresponds to the plane of the drawing, a deflection angle being controllable by rotating the first hand wheel 103. Further, the steerable section 10a can be bent in a second plane perpendicular to the first plane and perpendicular to the plane of the drawing, a corresponding deflection angle being controllable by rotating the second hand wheel 104. The surgical instrument 100 further exemplarily comprises a working channel, which is inserted into the shaft 10. The working channel is configured for communicating fluids or medical tools from the operating interface 102 to the distal end 11 of the shaft 10. Thereby, the working channel comprises a tubular body and an optical fiber. The tubular body has a main canal for receiving the fluids or medical tools and a secondary canal. The optical fiber is integrated in the secondary canal.

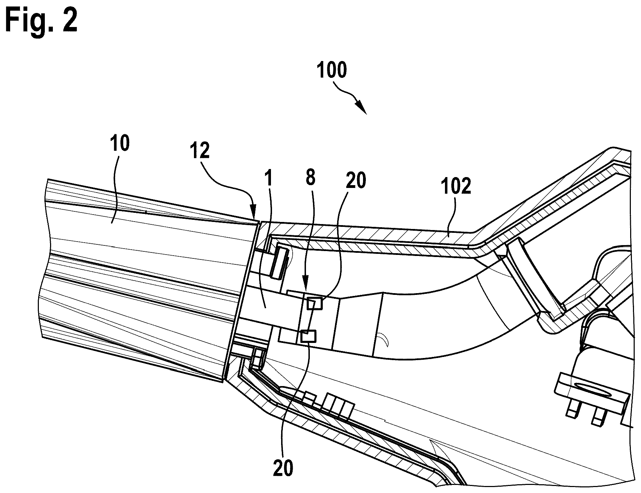

Fig. 2 shows a schematic cut view of an operating interface 102 of the surgical instrument 100 of Fig. 1.

Here, the surgical instrument 100 exemplarily comprises a shaft 10 with a distal end 11 and a proximal end 12 as well as a working channel 1, which is inserted into the shaft 10.

The working channel 1 is configured for communicating fluids or medical tools from the operating interface 102 to the distal end 11 of the shaft 10. Thereby, the working channel 1 comprises a tubular body and an optical fiber. The tubular body has a main canal for receiving the fluids or medical tools and a secondary canal. The optical fiber is integrated in the secondary canal.

Furthermore, the surgical instrument 100 contains a light source 20, which is connected to a proximal end 8 of the working channel 1 and transmits light to the optical fiber. In particular, the light source 20 can be directly connected to a proximal end of the optical fiber. The light source 20 can be configured as a LED. In Fig. 2 two LEDs 20 are arranged at the proximal end 8. For example, the number of LEDs 20 can correspond to the number of optical fibers.

Moreover, a distal end of the working channel 1 can be arranged flush with a distal end of the surgical instrument 100. Alternatively or additionally, the shaft 10 can be configured as a flexible shaft for being articulatable at least in one plain.

Further, the operating interface 102 can be arranged at the proximal end 12 of the shaft 10 and configured to operate the shaft 10. Furthermore, a light-adapter component can be arranged in between the optical fiber and the LED 20, in particular when the LED is larger than the optical fiber.

Additionally, the surgical instrument can comprise two steering wires (not shown) extending from the operating interface 102 through the flexible shaft 10 for articulating the flexible shaft.

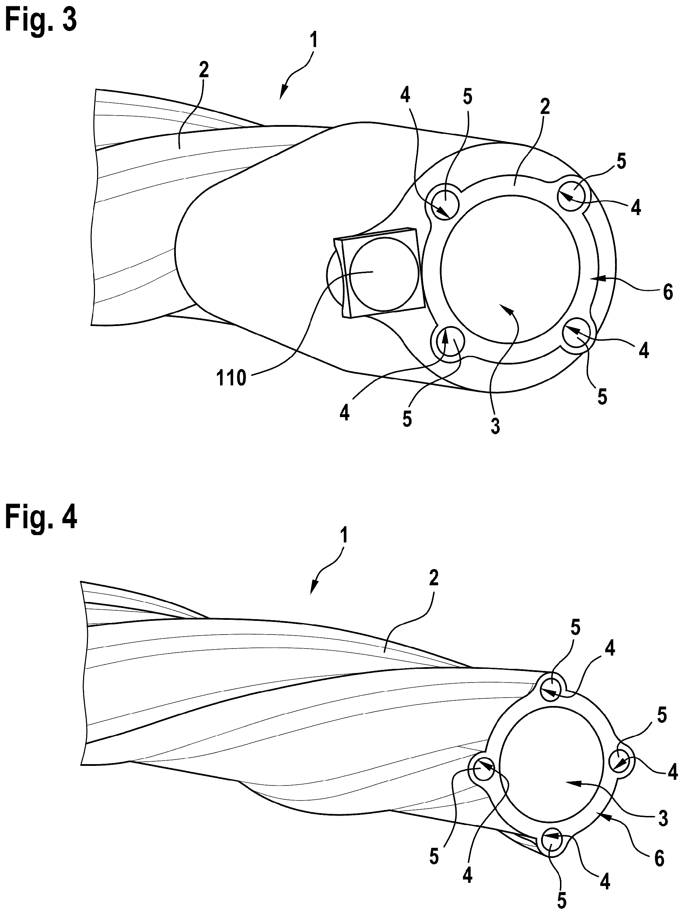

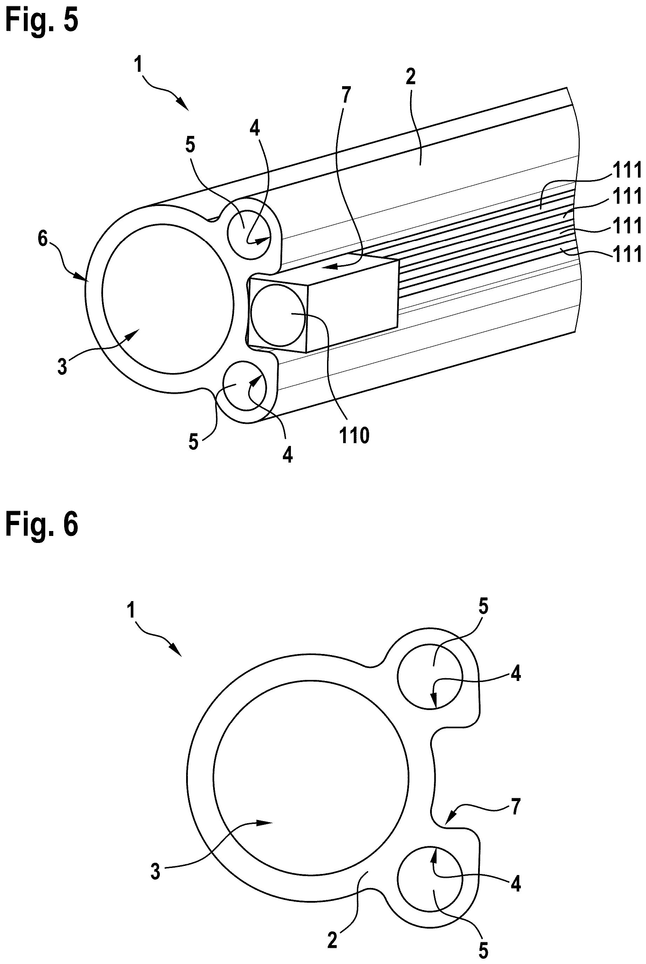

The surgical instrument 100 can further comprise an optical image device 110 arranged at the distal end 11, as it is illustrated in Figs. 3 and 5. The optical image device 110 or electronic cables assigned to it may extend from the operating interface 102 along the working channel 1 for generating image data of an area in front of the distal end.

Fig. 3 shows a schematic perspective view of a distal end 6 of a working channel 1 in combination with an optical image device 110 according to a further embodiment of the invention. Fig. 4 shows a schematic perspective view of the distal end 6 of the working channel 1 of Fig. 3.

The working channel 1 is configured for communicating fluids or medical tools through a shaft 10 of a surgical instrument 100, in particular of a flexible endoscopic instrument. Exemplarily the working channel 1 comprises a tubular body 2 and an optical fiber 5.

The tubular body 2 has a main canal 3 for receiving the fluids or medical tools and a secondary canal 4. Here, the secondary canal 4 comprises a plurality of secondary canals, for example four secondary canals 4.

The optical fiber 5 is integrated in the secondary canal 4. Here, the optical fiber 5 comprises a to the plurality of secondary canals corresponding plurality of optical fibers, for example four optical fibers 5. Each optical fiber 5 is integrated in one of the four secondary canals 4.

Exemplarily, the optical fibers 5 can be helically arranged around the main canal 3, as it is in particular illustrated in Fig. 4. The helical construction of the optical fiber 5 allows the working channel 1 to be more flexible or elastically deformable during its use. Additionally, light can be distributed in a wider field of view when the working channel 1 comprises the plurality of optical fibers 5 as each optical fiber 5 is directed at a different angle.

The working channel 1 can be manufactured in one manufacturing process, in particular in one extrusion process. Furthermore, the optical fibers 5 can be made of a glass material or of a transparent plastic material. The transparent plastic material can be polymethylmethacrylat, short PMMA, for example.

Optionally, each optical fiber 5 can have a cladding, that is configured to reflect and guide the light in the optical fiber 5. When embedded within the working channel 1, the tubular body 2 can protect the optical fibers 5 creating a very compact structure. The cladding can be arranged on an outer surface of the optical fiber 5, for example.

As it is shown in Fig. 3, the optical fibers 5 can be arranged flush with a distal end 6 of the tubular body 2. Additionally, the optical image device 110 can be arranged flush with the distal end 6. With this arrangement a fiber end of each optical fiber 5 can distribute light from the distal end 6 of the tubular body 2.

Optionally, the working channel 1 can house a combination of the optical fibers 5 and an electrical conductor embedded inside the working channel 1.

Fig. 5 shows a schematic perspective view of a distal end 6 of a working channel 1 according to a further embodiment of the invention. Fig. 6 shows a cut view of a cross-section of the working channel 1 of Fig. 5.

The working channel 1 of Figs. 5 and 6 substantially comprises the same features as the working channel of Figs. 3 and 4 unless described otherwise. Here, the secondary canal 4 comprises a two secondary canals 4 and the optical fiber 5 comprises two optical fibers 5, which are integrated in the two secondary canals 4.

An outer shell surface of the tubular body 2 exemplarily forms a groove 7. The groove 7 can extend parallel to the two secondary canals 4. In particular, the groove 7 can be formed between the two secondary canals 4, as it is in particular illustrated in Fig. 5. The groove 7 can be configured to secure a position of a camera head 110 and its electronic cables respectively data cables 111 along the working channel 1. For example, the groove 7 can have a rectangular shape (as shown in Fig. 6), a curved shape or combinations thereof.

In the detailed description above, various features have been combined in one or more examples in order to improve the rigorousness of the illustration. However, it should be clear in this case that the above description is of merely illustrative but in no way restrictive nature. It serves to cover all alternatives, modifications and equivalents of the various features and exemplary embodiments. Many other examples will be immediately and directly clear to a person skilled in the art on the basis of his knowledge in the art in consideration of the above description.

The exemplary embodiments have been chosen and described in order to be able to present the principles underlying the invention and their application possibilities in practice in the best possible way. As a result, those skilled in the art can optimally modify and utilize the invention and its various exemplary embodiments with regard to the intended purpose of use. In the claims and the description, the terms "including" and "having" are used as neutral linguistic concepts for the corresponding terms "comprising". Furthermore, use of the terms "a", "an" and "one" shall not in principle exclude the plurality of features and components described in this way.

While at least one exemplary embodiment of the present invention(s) is disclosed herein, it should be understood that modifications, substitutions and alternatives may be apparent to one of ordinary skill in the art and can be made without departing from the scope of this disclosure. This disclosure is intended to cover any adaptations or variations of the exemplary embodiment(s). In addition, in this disclosure, the terms "comprise" or "comprising" do not exclude other elements or steps, the terms "a" or "one" do not exclude a plural number, and the term "or" means either or both. Furthermore, characteristics or steps which have been described may also be used in combination with other characteristics or steps and in any order unless the disclosure or context suggests otherwise. This disclosure hereby incorporates by reference the complete disclosure of any patent or application from which it claims benefit or priority.

### REFERENCE LIST

- 1: working channel
- 2: tubular body
- 3: main canal
- 4: secondary canal
- 5: optical fiber
- 6: distal end of the working channel
- 7: groove
- 8: proximal end of the working channel
- 10: shaft
- 10a: steerable section
- 11: distal end of the shaft
- 12: proximal end of the shaft
- 20: light source
- 100: surgical instrument
- 101: handpiece
- 102: operating interface
- 103: first hand wheel
- 104: second hand wheel
- 105: knob
- 106: control buttons
- 107: connector
- 108: light cable
- 109: instrument port
- 110: optical image device
- 111: electronic cables

## Claims

1. Working channel (1) configured for communicating fluids or medical tools through a shaft (10) of a surgical instrument (100), in particular of a flexible endoscopic instrument, comprising:
a tubular body (2) with a main canal (3) for receiving the fluids or medical tools and with a secondary canal (4); and
an optical fiber (5), which is integrated in the secondary canal (4).

2. Working channel (1) according to claim 1,
**characterized in that**
the working channel (1) is manufactured in one manufacturing process, in particular in one extrusion process.

3. Working channel (1) according to claim 1 or 2,
**characterized in that**
the optical fiber (5) is made of a glass material or of a transparent plastic material.

4. Working channel (1) according to one of the preceding claims,
**characterized in that**
the optical fiber (5) has a cladding, that is configured to reflect and guide the light in the optical fiber (5).

5. Working channel (1) according to one of the preceding claims,
**characterized in that**
the optical fiber (5) is arranged flush with a distal end (6) of the tubular body (2).

6. Working channel (1) according to one of the preceding claims,
**characterized in that**
the secondary canal (4) comprises a plurality of secondary canals, and wherein the optical fiber (5) comprises a to that corresponding plurality of optical fibers, wherein each optical fiber (5) is integrated in one of the plurality of secondary canals (4).

7. Working channel (1) according to one of the preceding claims,
**characterized in that**
the optical fiber (5) is helically arranged around the main canal (3).

8. Working channel (1) according to one of the preceding claims,
**characterized in that**
an outer shell surface forms a groove (7), which extends parallel to the secondary canal (4).

9. Working channel (1) according to claim 6 and 8,
**characterized in that**
the groove (7) is formed between two secondary canals (4).

10. Surgical instrument (100), in particular flexible endoscopic instrument, comprising:
a shaft (10) with a distal end (11) and a proximal end (12);
a working channel (1) according to one of the preceding claims, which is insertable into the shaft (10); and
a light source (20), which is connected to a proximal end (8) of the working channel (1) and transmits light to the optical fiber (5).

11. Surgical instrument (100) according to claim 10,
**characterized in that**
a distal end (6) of the working channel is arranged flush with a distal end of the surgical instrument.

12. Surgical instrument (100) according to claim 10 or 11,
**characterized in that**
the shaft (10) is configured as a flexible shaft for being articulatable at least in one plain.

13. Surgical instrument (100) according to one of the claims 10 to 12, further comprising
an operating interface (102), which is arranged at the proximal end (12) of the shaft (10) and configured to operate the shaft (10).

14. Surgical instrument (100) according to claim 12 and 13,
further comprising
two steering wires extending from the operating interface (102) through the flexible shaft (10) for articulating the flexible shaft.

15. Surgical instrument (100) according to one of the claims 10 to 14,
**characterized in that**
the surgical instrument (100) further comprises an optical image device (110) arranged at the distal end (11) and extending from the operating interface (102) along the working channel (1) for generating image data of an area in front of the distal end (11).
